# EUROPEAN PATENT APPLICATION

(11) **EP 3 664 101 A1**
(43) Date of publication of application: **10.06.2020**
(21) Application number: 18210701.1
(22) Date of filing: 06.12.2018
(51) Int. Cl.: G16H 50/70, G16H 50/20

(54) **A COMPUTER-IMPLEMENTED METHOD AND AN APPARATUS FOR USE IN DETECTING MALINGERING BY A FIRST SUBJECT IN ONE OR MORE PHYSICAL AND/OR MENTAL FUNCTION TESTS**

(71) Applicant: Koninklijke Philips N.V., 5656 AG Eindhoven (NL)
(72) Inventor: DE VRIES, Jan Johannes Gerardus, 5656 AE Eindhoven (NL)
(74) Representative: de Haan, Poul Erik

(57) **Abstract**

According to an aspect, there is provided an apparatus (2) for use in detecting malingering by a first subject in one or more physical and/or mental function tests, the apparatus (2) comprising a memory unit (10) and a processing unit (8). The memory unit (10) is configured to store information relating to a multivariate probability distribution, MPD, for a plurality of parameters, with each parameter relating to a particular physical and/or mental function test in a set of physical and/or mental function tests, the MPD being determined from one or more population test results, and each population test result relating to the performance of one or more tests in the set by a respective subject in a population of subjects and comprising values for the parameters relating to the one or more tests performed by the subject. The processing unit (8) is configured to receive a first test result for the first subject relating to the performance of one or more of the tests in the set by the first subject, with the first test result comprising values for the parameters relating to the one or more tests performed by the first subject; determine an indication of whether the first subject was malingering by comparing the values for the parameters in the first test result to the MPD; and output the determined indication.

## Description

### FIELD OF THE INVENTION

The disclosure relates to the detection of malingering by a subject, and in particular to a computer-implemented method and apparatus for use in detecting malingering by a subject in one or more physical and/or mental function tests.

### BACKGROUND OF THE INVENTION

Clinical practice often requires the determination of the mental and/or physical capability of a subject (a person, an individual, a patient) such as to support a healthcare professional in the determination of a diagnostic or a rehabilitation plan of said person for instance.

Clinical neuropsychology is the field of determining the cognitive functioning of a person by requiring them to perform various validated neuropsychological tests and, for example, scoring their ability to perform these tests against norm scores (or normative data) that are representative for healthy individuals of a comparable demographic.

Assessing cognitive functioning is relevant for various patient groups including dementia, stroke, traumatic brain injury, multiple sclerosis, depression, epilepsy and schizophrenia as well as for screening healthy populations. Due to the ageing population and the rapidly increasing prevalence of some of these diseases, there is an increasing recognition of the importance of cognitive functioning and that it is necessary to be able to perform a neuropsychological assessment in an efficient and reproducible manner.

In current clinical practice, neuropsychological tests are typically either (i) paper-pencil tests, where the patient is asked to draw an object on paper or to complete a predefined task which has been printed on the paper; (ii) auditory verbal tests, where the patient is asked to recall facts, number sequences or word groups and from these responses a score is calculated; and (iii) motor tasks where the patient is asked to complete a task that involves a physical activity such completing a puzzle or finger tapping.

According to current standard procedures, for a full neuropsychological assessment there are typically 30-40 individual tests that may require up to 8 hours to be taken and analyzed by qualified healthcare professional(s).

Malingering is where a person intentionally underperforms in physical and/or mental function tests to fabricate or exaggerate symptoms of a physical condition and/or a mental condition. Malingering is a common issue with neuropsychological assessments as well as physical assessments.

It is assessed as important to recognize this intentional underperformance so that subjects who are trying to deceive their assessment(s) results or outcomes can be identified. For example, subjects with suspected traumatic brain injury may have an incentive to malinger due to a litigation case, where a poor performance in cognitive tests would help the subjects' case in court. The same applies where a subject has (or appears to have) a physical injury. Therefore, in addition to the cognitive tests for the actual cognitive assessment, there is often also a test added to detect whether people are genuinely attempting to perform the test at the best of their cognitive abilities. An example of such a test could be the F-score in the Minnesota Multiphasic Personality Inventory (MMPI).

A test, such as the MMPI which is intended for, or used to detect whether people are genuinely attempting to perform the test at the best of their cognitive and/or physical abilities is referred to as a malingering test and when someone is intentionally underperforming such a test they are referred to as a malingerer. In one survey, it was estimated that some degree of symptom exaggeration occurs in 39% of mild head injury cases, in 30% of disability assessments, and in 29% of personal injury cases.

The detection of malingering in neuropsychological tests (and other mental function tests) as well as physical function tests is therefore a very important aspect for both the clinical practitioner and the court of law. However, malingering is often very difficult to detect based on individual test performance, test scores or other test outputs and people may have very advanced strategies to voluntarily underperform. There are various publications that describe how detailed analysis of the responses or results to specific tests can yield more information to help identify a malingerer from amongst genuine performances. These include examining the type of errors and sequence location in a digit span, Memory Error Patterns (MEPs) in the Rey-O complex figure test, or the Trail Making Test (TMT) A/B ratio together with the individual TMT trial time. It is considered that inconsistency in performance levels between a subject's performance and report of impairment is the most common indicator of malingering.

One problem or drawback of known means for detecting malingering (whether in mental function or physical function tests) is that the complex patterns in the tests that have to be assessed in order to detect a malingerer cannot always be done reliably "on the fly" (i.e. as they happen) by the assessor. It is therefore typically only possible to do this retrospectively after the assessment has been completed, or by adding specific malingering tests to the test battery (i.e. a set of tests to be performed) which adds time to the assessment, and unnecessary burden to the healthcare system.

Another problem or drawback is the complexity of the analysis of the 30-40 tests to be able to establish that a subject is malingering. This analysis may show clear malingering in some tests but not in others.

Yet another problem or drawback is that, while it is typically not that difficult to obtain a data set of test results for people with a particular diagnosis, it is much more difficult to collect a data set of realistic examples of tests where the subject was malingering. This is due not only to the incident rate of malingering being low, but studies in which malingered test results are collected tend to be biased and do not reflect reality well.

### SUMMARY OF THE INVENTION

These problems are such that, among other things, current determination as if a subject is malingering (i) requires unnecessary resources since the neuropsychologist would probably have either abandoned the battery of tests had they known that the subject was malingering or perhaps even adapted the test battery to collect more evidence, and (ii) is time consuming and costly for the healthcare system to come to a conclusion.

It is an object of the present invention to provide improved apparatuses and methods to address one or more of the above problems such as to enable cost-effective, quick reliable and accurate detection as to whether a subject is malingering in one or more physical and/or mental function tests.

According to a first aspect, there is provided an apparatus for use in detecting malingering by a first subject in one or more physical and/or mental function tests. The apparatus comprises a memory unit configured to store information relating to a multivariate probability distribution, MPD, for a plurality of parameters, wherein each parameter relates to a particular physical and/or mental function test in a set of physical and/or mental function tests, wherein the MPD is determined from one or more population test results, wherein each population test result relates to the performance of one or more tests in the set by a respective subject in a population of subjects and comprises values for the parameters relating to the one or more tests performed by the subject. The apparatus further comprises a processing unit configured to: receive a first test result for the first subject relating to the performance of one or more of the tests in the set by the first subject, wherein the first test result comprises values for the parameters relating to the one or more tests performed by the first subject; determine an indication of whether the first subject was malingering by comparing the values for the parameters in the first test result to the MPD; and output the determined indication. Thus, the apparatus provides that malingering can be detected based on a set of population test results, without that set of population test results including any test results for subjects that were malingering. In addition, it is possible to determine an indication of whether the first subject was malingering once results for one or more tests are available for the first subject, so a malingering indication can be determined at a relatively early stage of a battery of tests. This malingering indication can be refined as more test results become available for the first subject.

In some embodiments, two or more of the plurality of parameters relate to the same test.

In some embodiments, one or more of the population test results relate to the performance of a plurality of tests in the set by a respective subject. These embodiments enable a single malingering indication to be determined based on the results of a number of different tests performed by the first subject, which is useful in the case where, for example, an individual analysis of the test results might show clear malingering in some tests, but not in others.

In some embodiments, each of the population test results relate to the performance of a plurality of tests in the set by a respective subject. These embodiments allow for the population test results to relate to different combinations of tests by the subjects in the population (i.e. it is not necessary for each of the subjects in the population to have completed exactly the same set of tests), which makes gathering the population test results more straightforward.

In some embodiments, the first test result relates to the performance of a plurality of tests in the set by the first subject.

In some embodiments, the processing unit is configured to determine an indication of whether the first subject was malingering by determining a probability value from the MPD corresponding to the values for the parameters in the first test result, wherein the probability value indicates a likelihood of observing the first test result in the population of subjects. These embodiments provide a simple indication of the likelihood that the first subject was malingering, or, put another way, a simple indication of whether the first subject is showing behavior that was not observed in the population of subjects. In these embodiments, the processing unit can be configured to determine the indication of whether the first subject was malingering by comparing the determined probability value to a first threshold; and determining an indication that the first subject was malingering if the determined probability value is below the first threshold.

In some embodiments, the stored information relating to the MDF is a set of novelty scores for the plurality of parameters determined from the MPD, and wherein the processing unit is configured to determine an indication of whether the first subject was malingering by determining a novelty score from the set of novelty scores corresponding to the values for the parameters in the first test result. These embodiments also provide a simple indication of the likelihood that the first subject was malingering, or, put another way, a simple indication of whether the first subject is showing behavior that was not observed in the population of subjects. In these embodiments, the processing unit can be configured to determine the indication of whether the first subject was malingering by comparing the determined novelty score to a second threshold; and determining an indication that the first subject was malingering if the determined novelty score is above the second threshold.

In some embodiments, the MPD is a multivariate probability distribution function or a multivariate probability density function.

In some embodiments, the processing unit is further configured to receive the one or more population test results; and determine the MPD from the one or more population test results. In these embodiments, the processing unit can be configured to determine the MPD from the one or more population test results by forming an N-dimensional histogram from the population test results, where N is the number of parameters in the plurality of parameters. In these embodiments, the processing unit can be configured to determine the MPD from the one or more population test results using a mixture model. These embodiments have the advantage that the MPD is smoother and allows for better estimations of the malingering indication when small numbers of population test results are used to form the MPD. In addition, the mixture model interpolates between and extrapolates values in the population test results, hence allowing for better comparison with parameter values that were not seen in the population test results. In some embodiments, the processing unit can be configured to determine the MPD from the one or more population test results by determining a plurality of sub-MPDs from respective subsets of the population test results, wherein each subset of population test results comprises population test results for subjects having a similar health status; and combining the plurality of sub-MPDs to form the MPD. In these embodiments, the health status for a first subset of the population test results can be 'healthy', and the health status for a second subset of the population test results can be one or more physical and/or mental impairments or conditions.

According to a second aspect, there is provided a computer-implemented method for detecting malingering by a first subject in one or more physical and/or mental function tests. The method comprises: storing, in a memory unit, information relating to a multivariate probability distribution, MPD, for a plurality of parameters, wherein each parameter relates to a particular physical and/or mental function test in a set of physical and/or mental function tests, wherein the MPD is determined from one or more population test results, wherein each population test result relates to the performance of one or more tests in the set by a respective subject in a population of subjects and comprises values for the parameters relating to the one or more tests performed by the subject; receiving, at a processing unit, a first test result for the first subject relating to the performance of one or more of the tests in the set by the first subject, wherein the first test result comprises values for the parameters relating to the one or more tests performed by the first subject; determining, by the processing unit, an indication of whether the first subject was malingering by comparing the values for the parameters in the first test result to the MPD; and outputting, by the processing unit, the determined indication. Thus, the method provides that malingering can be detected based on a set of population test results, without that set of population test results including any test results for subjects that were malingering. In addition, it is possible to determine an indication of whether the first subject was malingering once results for one or more tests are available for the first subject, so a malingering indication can be determined at a relatively early stage of a battery of tests. This malingering indication can be refined as more test results become available for the first subject.

In some embodiments, two or more of the plurality of parameters relate to the same test.

In some embodiments, one or more of the population test results relate to the performance of a plurality of tests in the set by a respective subject. These embodiments enable a single malingering indication to be determined based on the results of a number of different tests performed by the first subject, which is useful in the case where, for example, an individual analysis of the test results might show clear malingering in some tests, but not in others.

In some embodiments, each of the population test results relate to the performance of a plurality of tests in the set by a respective subject. These embodiments allow for the population test results to relate to different combinations of tests by the subjects in the population (i.e. it is not necessary for each of the subjects in the population to have completed exactly the same set of tests), which makes gathering the population test results more straightforward.

In some embodiments, the first test result relates to the performance of a plurality of tests in the set by the first subject.

In some embodiments, the step of determining an indication of whether the first subject was malingering comprises determining a probability value from the MPD corresponding to the values for the parameters in the first test result, wherein the probability value indicates a likelihood of observing the first test result in the population of subjects. These embodiments provide a simple indication of the likelihood that the first subject was malingering, or, put another way, a simple indication of whether the first subject is showing behavior that was not observed in the population of subjects. In these embodiments, the step of determining the indication of whether the first subject was malingering can comprise comparing the determined probability value to a first threshold; and determining an indication that the first subject was malingering if the determined probability value is below the first threshold.

In some embodiments, the information relating to the MDF stored in the step of storing is a set of novelty scores for the plurality of parameters determined from the MPD, and wherein the step of determining an indication of whether the first subject was malingering comprises determining a novelty score from the set of novelty scores corresponding to the values for the parameters in the first test result. These embodiments also provide a simple indication of the likelihood that the first subject was malingering, or, put another way, a simple indication of whether the first subject is showing behavior that was not observed in the population of subjects. In these embodiments, the step of determining the indication of whether the first subject was malingering can comprise comparing the determined novelty score to a second threshold; and determining an indication that the first subject was malingering if the determined novelty score is above the second threshold.

In some embodiments, the MPD is a multivariate probability distribution function or a multivariate probability density function.

In some embodiments, the method further comprises the steps of: receiving, at the processing unit, the one or more population test results; and determining, by the processing unit, the MPD from the one or more population test results. In these embodiments, the step of determining the MPD from the one or more population test results can comprise forming an N-dimensional histogram from the population test results, where N is the number of parameters in the plurality of parameters. In these embodiments, the step of determining the MPD from the one or more population test results comprises using a mixture model. These embodiments have the advantage that the MPD is smoother and allows for better estimations of the malingering indication when small numbers of population test results are used to form the MPD. In addition, the mixture model interpolates between and extrapolates values in the population test results, hence allowing for better comparison with parameter values that were not seen in the population test results. In some embodiments, the step of determining the MPD from the one or more population test results comprises determining a plurality of sub-MPDs from respective subsets of the population test results, wherein each subset of population test results comprises population test results for subjects having a similar health status; and combining the plurality of sub-MPDs to form the MPD. In these embodiments, the health status for a first subset of the population test results can be 'healthy', and the health status for a second subset of the population test results can be one or more physical and/or mental impairments or conditions.

According to a third aspect, there is provided a computer program product comprising a computer readable medium having computer readable code embodied therein, the computer readable code being configured such that, on execution by a suitable computer having a memory unit, or processor having a memory unit associated therewith, the computer or processor is caused to perform the method according to the second aspect or any embodiment thereof.

These and other aspects will be apparent from and elucidated with reference to the embodiment(s) described hereinafter.

### BRIEF DESCRIPTION OF THE DRAWINGS

Exemplary embodiments will now be described, by way of example only, with reference to the following drawings, in which:
Fig. 1 is a block diagram illustrating an apparatus according to an exemplary embodiment;
Fig. 2 is a flow chart illustrating a method according to an exemplary embodiment;
Fig. 3 is an illustration of an exemplary multivariate probability distribution in the form of a histogram;
Fig. 4 is an illustration of an exemplary multivariate probability distribution formed using a Gaussian mixture model;
Fig. 5 is an illustration of an alternative exemplary multivariate probability distribution formed using a Gaussian mixture model; and
Fig. 6 is an illustration of an exemplary multivariate probability distribution that provides novelty scores.

### DETAILED DESCRIPTION OF EMBODIMENTS

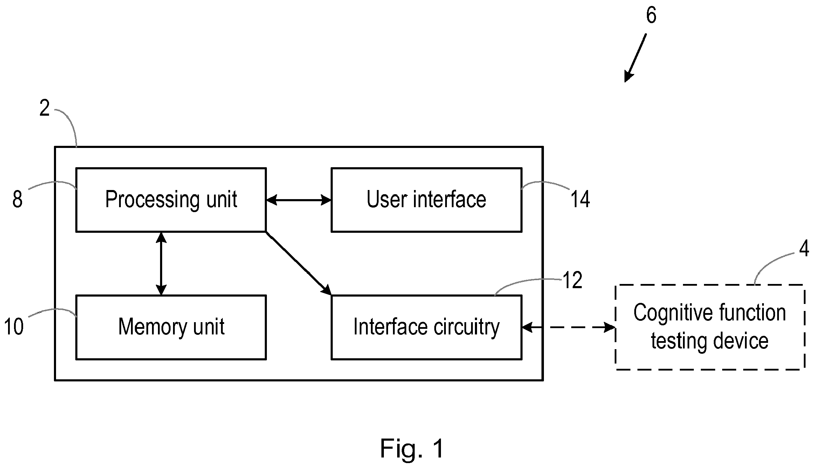

Fig. 1 shows a block diagram of an exemplary apparatus 2 that can be used in detecting malingering by a subject in tests of physical and/or mental function of the subject. It should be noted that the 'subject' referenced herein is the person or individual that is taking part in the activity or test. In some embodiments, the apparatus 2 can also be used by the subject to perform or complete the one or more tests of physical and/or mental function. As such the apparatus 2 may be, or may comprise, a testing device for providing the physical and/or mental function tests. Alternatively, the apparatus 2 may be configured to be connected to a testing device 4 that provides the one or more physical and/or mental function tests to form a system 6 for detecting malingering by a subject during the physical and/or mental function testing.

The testing device 4 (or the apparatus 2 in the embodiments where the apparatus 2 is used to provide the one or more physical and/or mental function tests) can comprise a device or component that is used store physical and/or mental function test material, that can output required physical and/or mental function test material to a subject or to a separate device belonging to or used by the subject (such as a computer, tablet, smartphone, television, etc.), and that is used to receive and analyze the results of the inputs by the subject in response to the presented test. In some embodiments, the testing device 4 can be self-contained device (e.g. a computer, laptop, tablet computer, smart watch, smartphone, etc.) that stores the physical and/or mental function test material, presents the test to the subject (e.g. via a display screen), receives the inputs by the subject and analyses the results to provide a measure of the performance of the subject in the test. In some other embodiments, the testing device 4 can be a device or component (e.g. a computer, server, etc.) that stores the cognitive function test material, outputs the test material to a display device (e.g. a screen, a television, a smart phone, etc.), receives information representing the subject's inputs in response to the presented test and analyses the results to provide a measure of the performance of the subject in the test. In these embodiments, the testing device 4 can be a server that provides the test material to a web browser or application on the other device, and the subject's inputs returned to the server via the web browser or application for analysis and evaluation. It will be appreciated that the above embodiments also apply where the apparatus 2 is used to provide the cognitive function tests.

The apparatus 2 is an electronic device that comprises a processing unit 8 and a memory unit 10. The processing unit 8 is configured or adapted to control the operation of the apparatus 2 and to implement the techniques described herein for detecting malingering by a subject.

The processing unit 8 can be configured to execute or perform the methods described herein. The processing unit 8 can be implemented in numerous ways, with software and/or hardware, to perform the various functions described herein. The processing unit 8 may comprise one or more microprocessors or digital signal processor (DSPs) that may be programmed using software or computer program code to perform the required functions and/or to control components of the processing unit 8 to effect the required functions. The processing unit 8 may be implemented as a combination of dedicated hardware to perform some functions (e.g. amplifiers, pre-amplifiers, analog-to-digital converters (ADCs) and/or digital-to-analog convertors (DACs)) and a processor (e.g., one or more programmed microprocessors, controllers, DSPs and associated circuitry) to perform other functions. Examples of components that may be employed in various embodiments of the present disclosure include, but are not limited to, conventional microprocessors, DSPs, application specific integrated circuits (ASICs), and field-programmable gate arrays (FPGAs).

The processing unit 8 is connected to a memory unit 10 that can store data, information and/or signals for use by the processing unit 8 in controlling the operation of the apparatus 2 and/or in executing or performing the methods described herein. In some implementations the memory unit 10 stores computer-readable code that can be executed by the processing unit 8 so that the processing unit 8, in conjunction with the memory unit 10, performs one or more functions, including the methods described herein. The memory unit 10 can comprise any type of non-transitory machine-readable medium, such as cache or system memory including volatile and non-volatile computer memory such as random access memory (RAM), static RAM (SRAM), dynamic RAM (DRAM), read-only memory (ROM), programmable ROM (PROM), erasable PROM (EPROM), and electrically erasable PROM (EEPROM), and the memory unit 10 can be implemented in the form of a memory chip, an optical disk (such as a compact disc (CD), a digital versatile disc (DVD) or a Blu-Ray disc), a hard disk, a tape storage solution, or a solid state device, including a memory stick, a solid state drive (SSD), a memory card, etc.

The apparatus 2 also includes interface circuitry 12 for enabling a data connection to and/or data exchange with other devices, including any one or more of servers, databases, user devices, and one or more testing devices 4. The connection may be direct or indirect (e.g. via the Internet), and thus the interface circuitry 12 can enable a connection between the apparatus 2 and a network, such as the Internet, via any desirable wired or wireless communication protocol. For example, the interface circuitry 12 can operate using WiFi, Bluetooth, Zigbee, or any cellular communication protocol (including but not limited to Global System for Mobile Communications (GSM), Universal Mobile Telecommunications System (UMTS), Long Term Evolution (LTE), LTE-Advanced, etc.). In the case of a wireless connection, the interface circuitry 12 (and thus apparatus 2) may include one or more suitable antennas for transmitting/receiving over a transmission medium (e.g. the air). Alternatively, in the case of a wireless connection, the interface circuitry 12 may include means (e.g. a connector or plug) to enable the interface circuitry 12 to be connected to one or more suitable antennas external to the apparatus 2 for transmitting/receiving over a transmission medium (e.g. the air). The interface circuitry 12 is connected to the processing unit 8.

In some embodiments, the apparatus 2 comprises a user interface 14 that includes one or more components that enables a user of apparatus 2 to input information, data and/or commands into the apparatus 2, and/or enables the apparatus 2 to output information or data to the user of the apparatus 2. As used herein, the 'user' of the apparatus can be a person, such as a neuropsychologist, that would like to determine if a test subject (referred to as a 'subject' or 'first subject' herein) is malingering. In embodiments where the apparatus 2 includes or is part of a testing device, the subject can also be considered as a user of the apparatus 2.

The user interface 14 can comprise any suitable input component(s), including but not limited to a keyboard, keypad, one or more buttons, switches or dials, a mouse, a track pad, a touchscreen, a stylus, a camera, a microphone, etc., and the user interface 14 can comprise any suitable output component(s), including but not limited to a display screen, one or more lights or light elements, one or more loudspeakers, a vibrating element, etc.

The apparatus 2 can be any type of electronic device or computing device. For example the apparatus 2 can be, or be part of, a server, a computer, a laptop, a tablet, a smartphone, a smartwatch, etc. In some implementations, for example where the apparatus 2 is also used as the test device to present the test(s) to the subject, the apparatus 2 can be an apparatus that is present or used in the home or care environment of the subject/user. In other implementations, the apparatus 2 is an apparatus that is remote from the first subject, and remote from the home or care environment of the first subject.

It will be appreciated that a practical implementation of an apparatus 2 may include additional components to those shown in Fig. 1. For example the apparatus 2 may also include a power supply, such as a battery, or components for enabling the apparatus 2 to be connected to a mains power supply.

As noted above, problems in detecting malingering in physical and/or mental function tests include that the complex patterns in the tests that have to be assessed in order to detect a malingerer cannot always be done reliably "on the fly" (i.e. as they happen) by the assessor, the analysis of the 30-40 tests typically required to be able to establish that a subject is malingering is complex (with the analysis often showing clear malingering in some tests but not in others) and, while it is typically not that difficult to obtain a data set of test results for people with a particular diagnosis, it is much more difficult to collect a data set of realistic examples of tests where the subject was malingering.

As used herein, a "physical function test" or a "test of physical function" can test any aspect of physical function of a subject, including the gross motor skills/ability of the subject (e.g. ability to walk steadily, lift objects, etc.) and the fine motor skills/ability of the subject (e.g. relating to the functioning of the hands or fingers). Also as used herein, a "mental function test" or a "test of mental function" can test any aspect of mental function of a subject, including the cognitive functions of the subject, such as memory, perception, attention, motor skills, language, visual and spatial processing and executive functions. A physical and/or mental function test can include one or more activities that the subject is to perform and/or one or more questions that the subject is to answer.

In view of the problems in detecting malingering, the techniques described herein therefore provide an approach by which a multivariate probability distribution (MPD) is provided for a plurality of parameters which each relate to a particular physical and/or mental function test. The MPD is formed from results for a population of subjects who have performed one or more physical and/or mental function tests from a set of available physical and/or mental function tests, with the population results including test results from subjects that are healthy, as well as from subjects that have one or more medical conditions that can affect or impair their physical and/or mental functions. In view of the way in which the MPD is used to identify malingerers, the population test results preferably do not include results for subjects that malingered when performing the tests. However, it is not required for the population test results to exclude malingerers, only that the occurrence of malingering in the population test results is small relative to non-malingering test results.

For a given set of test results by a first subject (with the test results being provided in the form of values for parameters relating to those tests), the MPD can provide a value that indicates the probability, likelihood or similar indication of observing that set of test results in the population. Provided that the population test results do not include any test results for subjects that were malingering, or that were malingering in the particular set of tests by the first subject (or that were malingering in the same way as the first subject, if the first subject was malingering), the value provided by the MPD will be indication of whether the first subject was malingering.

Thus, to determine if a first subject may be malingering, when test results are received for the first subject, the values for the parameters in the test results are compared to the MPD to determine an indication of whether the first subject was malingering, and this indication can be output in some form. For example the indication can be output to a user of the apparatus 2 (e.g. a neuropsychologist), and/or the indication can be output to a database, other apparatus or system that can analyze, report and/or store the malingering indication.

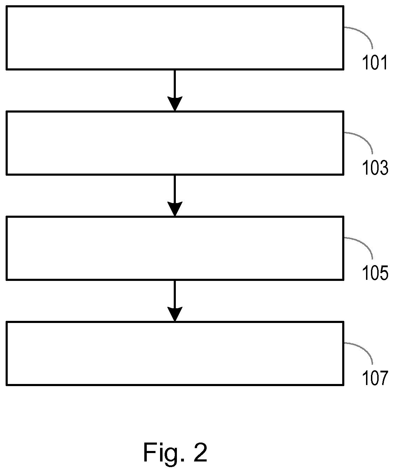

The flow chart in Fig. 2 shows an exemplary method for detecting malingering by a first subject according to various embodiments. Some or all of the steps in the method can be performed by the apparatus 2, for example by the processing unit 8 in conjunction with the memory unit 10. In that case, the processing unit 8 can be configured to perform the method, and/or the processing unit 8 can execute computer program code to cause the processing unit 8 to perform the method.

In step 101, information relating to a multivariate probability distribution (MPD) is stored, for example in memory unit 10. The MPD is for a plurality of parameters, with each parameter relating to an aspect of the performance of a particular physical and/or mental function test. The MPD provides a value representing the probability, likelihood or similar of observing a particular set of values for the parameters compared to the population test results used to form the MPD.

There is a set of physical and/or mental function tests, which includes one or more (but preferably a plurality of) physical and/or mental function tests. A set of physical and/or mental function tests can also be referred to as a 'battery' of tests.

Multiple parameters can relate to (or be derived from) a particular test. For example, a particular physical and/or mental function test may test the speed and accuracy with which a subject completes a series of tasks (e.g. hitting or cancelling targets), and thus the parameters relating to that test can include the time taken to complete the test and the percentage accuracy.

Preferably, the plurality of parameters that the MPD relates to are for a plurality of different physical and/or mental function tests (with the possibility that multiple parameters can relate to a particular physical and/or mental function test), as this enables the test results for a subject for a number of different physical and/or mental function tests to be assessed for malingering together.

The MPD is determined from one or more population test results, with each population test result relating to the performance of one or more tests in the set by a respective subject in a population of subjects. Each population test result comprises values for the parameters relating to the one or more tests performed by the subject. Preferably, most or all of the population test results relate to a plurality of tests performed by the respect subject in the population. However, it will be appreciated that it is not necessary for each population test result to comprise values for all of the available parameters (in other words it is not necessary for each population test result to include results for all of the available sets), and indeed different population test results can relate to different combinations of physical and/or mental function tests.

As noted above, the population of subjects includes subjects that are healthy, for example subjects that do not have any conditions that impair their physical functions or mental functions, and that would otherwise impair their performance in a physical function test or in a mental function test. The population of subjects also includes subjects that have one or more medical conditions that impair their physical functions and/or mental functions, and that would impair their performance in one or more physical function tests and/or in one or more mental function tests. For example the population of subjects can include one or more subjects that have suffered a stroke, and/or one or more subjects that have suffered a traumatic brain injury. The subjects in the population can have a range of different medical conditions. In view of the way in which the MPD is used to identify malingerers, the population test results preferably do not include results for subjects that malingered when performing the tests. However, it is not required for the population test results to exclude malingerers, only that the occurrence of malingering in the population test results is small.

In step 103, test results for the first subject are received. These test results are referred to herein as the "first test result", and relate to the performance of one or more of the tests in the set by the first subject. As such, the first test result comprises values for the parameters relating to the one or more tests performed by the first subject. In some embodiments, the first test result relates to the performance of a plurality of the tests in the set by the first subject.

The first test results can be received by the processing unit 8. In some embodiments, the processing unit 8 can receive the first test results via the interface circuitry 12 from a test device 4 or from an external test result storage unit. In other embodiments, the processing unit 8 can receive the first test results by retrieving the first test results from the memory unit 10.

Next, in step 105, the processing unit 8 determines an indication of whether the first subject was malingering by comparing the values for the parameters in the first test result to the MPD.

In step 107 the determined indication is output by the processing unit 8. Step 107 can comprise outputting the determined indication to a user of the apparatus 2 (e.g. a neuropsychologist). In this case the indication can be output using the user interface 14 (e.g. displaying the indication on a display screen). Step 107 can also or alternatively comprise outputting the indication to, and storing the indication in, the memory unit 10, a database, or other apparatus or system that can analyze, report and/or store the malingering indication.

In some embodiments, the MPD is a multivariate probability distribution function or a multivariate probability density function. It will be appreciated that the MPD can be a multivariate probability distribution function where the values for the parameters are discrete values from a set of possible values, and the MPD can be a multivariate probability density function where the values for the parameters are values from a continuous range of possible values.

In either case, step 105 can comprise determining a probability value from the MPD corresponding to the values for the parameters in the first test result. The probability value (which is also referred to as a likelihood value) indicates a likelihood of observing the values of the parameters in the first test result (i.e. the values taken as a group) in the population of subjects.

In some embodiments, the probability value can be output in step 107. In alternative embodiments, step 105 can further comprise comparing the determined probability value to a first threshold, and determining the indication from the result of the comparison. In particular, as the population test results do not include test results where the subject was malingering (or the population test results include relatively few test results where the subject was malingering), the probability value will be higher if the first test results are consistent with the subject being healthy or being impaired in some way due to a medical condition (i.e. if the first test results are consistent with non-malingering test results), and the probability value will be lower if the first test results are not consistent with the subject being healthy or being impaired in some way due to a medical condition (i.e. if the first test results are not consistent with non-malingering test results). The first threshold can have a value that is suitable to distinguish between high probability values associated with genuine test behavior, and low probability values associated with malingering test behavior. In these embodiments, the indication of whether the first subject was malingering can be the result of the comparison of the probability value to the first threshold (e.g. 'above' or 'below' the first threshold). Alternatively, based on the comparison to the first threshold, the indication can be, e.g. 'malingering' or 'not malingering'/'genuine'.

In some embodiments, a so-called 'novelty score distribution' can be derived from the MPD which provides a set of novelty scores for the plurality of parameters. A technique for deriving a novelty score distribution is described further below, but approximately, a novelty score for a first test result is the inverse of a probability value from the MPD, and thus the magnitude of the novelty score for the first test result indicates how 'new' that first test result is amongst the population test results used to determine the MPD. Thus, in step 105 a novelty score can be determined from the set of novelty scores that corresponds to the values for the parameters in the first test result.

In some embodiments, the novelty score can be output in step 107. In alternative embodiments, step 105 can further comprise comparing the determined novelty score to a second threshold, and determining the indication from the result of the comparison. In particular, as the population test results do not include test results where the subject was malingering (or the population test results include relatively few test results where the subject was malingering), the novelty score will be lower if the first test results are consistent with the subject being healthy or being impaired in some way due to a medical condition (i.e. if the first test results are consistent with non-malingering test results), and the novelty score will be lower if the first test results are consistent with the subject being healthy or being impaired in some way due to a medical condition (i.e. if the first test results are not consistent with non-malingering test results). The second threshold can have a value that is suitable to distinguish between low novelty scores associated with genuine test behavior, and high novelty scores associated with malingering test behavior. In these embodiments, the indication of whether the first subject was malingering can be the result of the comparison of the novelty score to the second threshold (e.g. 'above' or 'below' the second threshold). Alternatively, based on the comparison to the second threshold, the indication can be, e.g. 'malingering' or 'not malingering'/'genuine'.

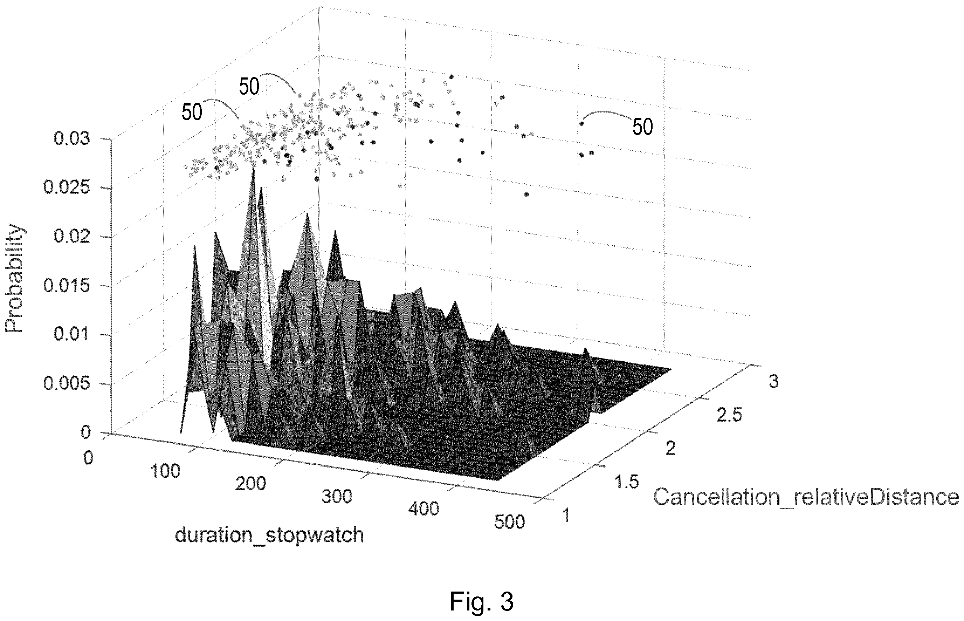

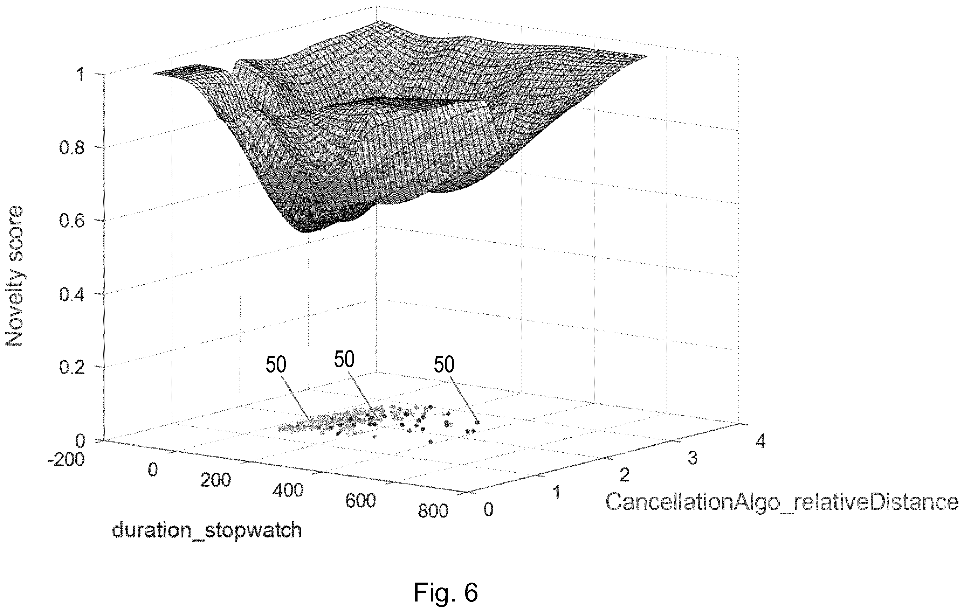

In some embodiments, prior to step 101, the method can further comprise a step of receiving the one or more population test results, followed by a step of determining the MPD from the one or more population test results. These steps can be performed by the processing unit 8, but it will be appreciated that these steps could be performed by a separate apparatus or device to apparatus 2. Embodiments of determining the MPD from the population test results are described in more detail below with reference to Figs. 3-6.

However, briefly, in some embodiments, the MPD can be determined from the population test results by forming an N-dimensional histogram from the population test results, where N is the number of parameters in the plurality of parameters. For example, for each of the N parameters, the range of possible values for the parameter is considered, and the range divided into a plurality of bins (e.g. 10 bins per parameter, although a different number of bins can be used, and each parameter can have a different number of bins). The bins for the N parameters are considered together which creates a set of N-dimensional bins, with each N-dimensional bin corresponding to a particular combination of values for the N parameters. Each population test result is put into the N-dimensional bin appropriate for the parameter values for that population test result. Once all population test results have been put into the relevant bin, the number of test results in each bin is counted, and this count forms the histogram.

In some other embodiments, the MPD can be determined from the population test results using a mixture model, such as a Gaussian mixture model. In some other embodiments, the MPD can be determined from the population test results by determining a plurality of sub-MPDs from respective subsets of the population test results and combining the plurality of sub-MPDs to form the MPD. In this case, each subset of population test results comprises population test results for subjects having a similar health status. For example, a subset of the population test results can relate to subjects that are healthy (i.e. have no medical condition, or no medical condition that affects their physical and/or mental function). Another subset of the population test results can relate to subjects having one or more physical and/or mental impairments or medical conditions (e.g. one subset can relate to the test results for subjects that have suffered a stroke, and another subset can relate to the test results for subjects that have Alzheimer's disease).

Thus, in some embodiments, to determine the MPD, a database of population test results is provided. Each population test result may be labelled with the diagnosis of the subject that took the tests. For example, the groups of subjects represented in this database could include healthy, subject who have suffered a stroke, subject who have suffered a traumatic brain injury, etc. The database of population test results can be used to determine the MPD, i.e. a model that represents the probability distribution of observed values.

Embodiments of the MPD that can be determined from the population test results are shown in Figs. 3-6. In each of these examples, the MPD is determined from population test results were obtained for a population of 241 healthy elderly subjects and 40 subjects who have suffered a stroke, with each population test result comprising values for two parameters relating to a particular physical and/or mental function test in which a subject has to cancel a series of targets by drawing a line through each of the targets in turn as quickly as possible and using as short a line as possible. The first parameter is denoted "duration_stopwatch" and represents the total time taken by the subject to cancel all of the targets, and the second parameter is denoted "CancellationAlgo relativeDistance" and represents the length of the line drawn by the subject to cancel all the targets relative to an optimal (shortest possible) line length. For both parameters, higher values (so a longer duration and longer line length) are associated with the subject having a physical and/or mental dysfunction. It will be appreciated that although the MPDs described below and shown in Figs. 3-6 are 2-dimensional (as they only relate to two parameters), the techniques described below are readily applicable to higher-dimensional MPDs (i.e. for more than two parameters).

In some embodiments, as mentioned above, an estimate for the underlying probability distribution can be a 2-dimensional histogram formed from the values of the parameters in the population test results. Fig. 3 shows an exemplary MPD in the form of a 2-D histogram, with respective axes for "duration stopwatch" and "CancellationAlgo_relativeDistance", and the vertical axis representing the probability of observing that combination of parameter values, based on the distribution of parameter values in the population test results. Fig. 3 shows data points 50 that each represent the parameter values for a particular test result in the population test results. However, it will be appreciated that these data points are only included in the graph for ease of understanding, and they are not part of the MPD itself. It can be seen from the distribution shown in Fig. 3 that a test result having a small value for "duration_stopwatch" (e.g. less than 200) and an intermediate value for "CancellationAlgo_relativeDistance" (e.g. around 2) has the highest probability (i.e. indicating that this combination of parameter values is likely to occur in the population of subjects, implying that the test result is genuine), whereas a test result having, say, a value of 350 for "duration_stopwatch" and a value of 1.2 for "CancellationAlgo_relativeDistance" has a zero probability as it was not seen in the population of subjects, and this can imply that the test result is the result of the subject malingering.

As noted above, a threshold (the first threshold) can be used to identify whether a probability value corresponding to a test result that is read out from the histogram is indicative of malingering.

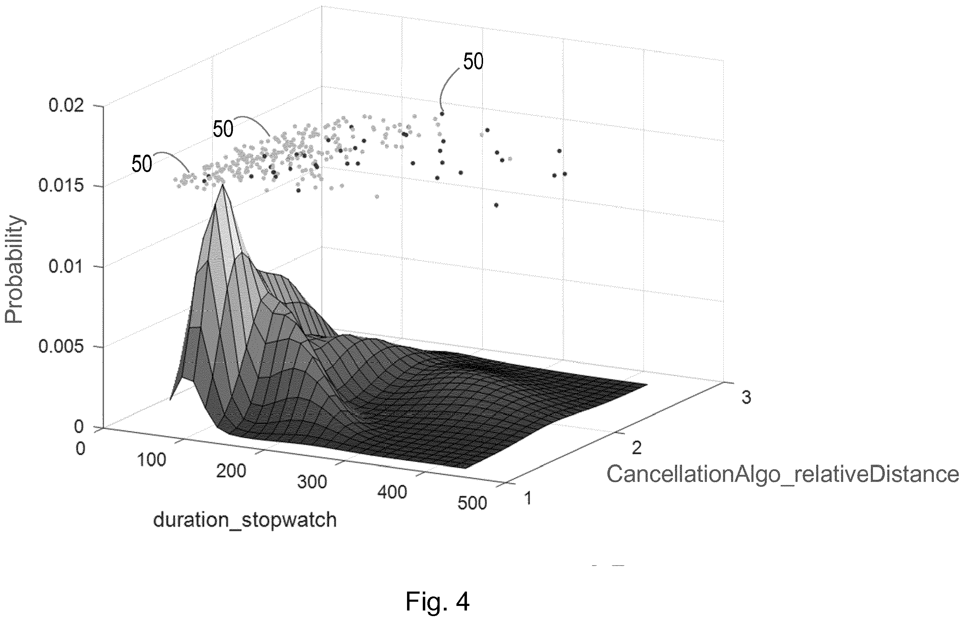

As an alternative to forming a histogram, the MPD can be a probability distribution function that is estimated as a mixture model, such as a Gaussian Mixture Model (GMM). Techniques for determining a probability distribution function as a mixture model from population test results are known in the art, and further details are not provided herein. However, the mixture model interpolates between and extrapolates values in the population test results, which allows for better comparison with parameter values that were not seen in the population test results. Fig. 4 shows an exemplary MPD formed using a Gaussian mixture model, based on the same population test data as in Fig. 3. Again, data points 50 are shown for ease of understanding. The use of a mixture model, and particularly a GMM, is advantageous as it results in a much smoother probability distribution (cf. the histogram approach in Fig. 3), and thus allows for better estimations of the malingering indication when small amounts of population test results are used to form the MPD.

As in the histogram example in Fig. 3, it can be seen from Fig. 4 that a test result having a small value for "duration_stopwatch" (e.g. less than 200) and an intermediate value for "CancellationAlgo_relativeDistance" (e.g. around 2) has the highest probability (i.e. indicating that this combination of parameter values is likely to occur in the population of subjects, implying that the test result is genuine), whereas a test result having, say, a value of 350 for "duration_stopwatch" and a value of 1.2 for "CancellationAlgo_relativeDistance" has a zero, or near-zero probability as it was not seen in the population of subjects, and this can imply that the test result is the result of the subject malingering. Again, a threshold (the first threshold) can be used to identify whether a probability value corresponding to a test result that is read out from the probability distribution is indicative of malingering.

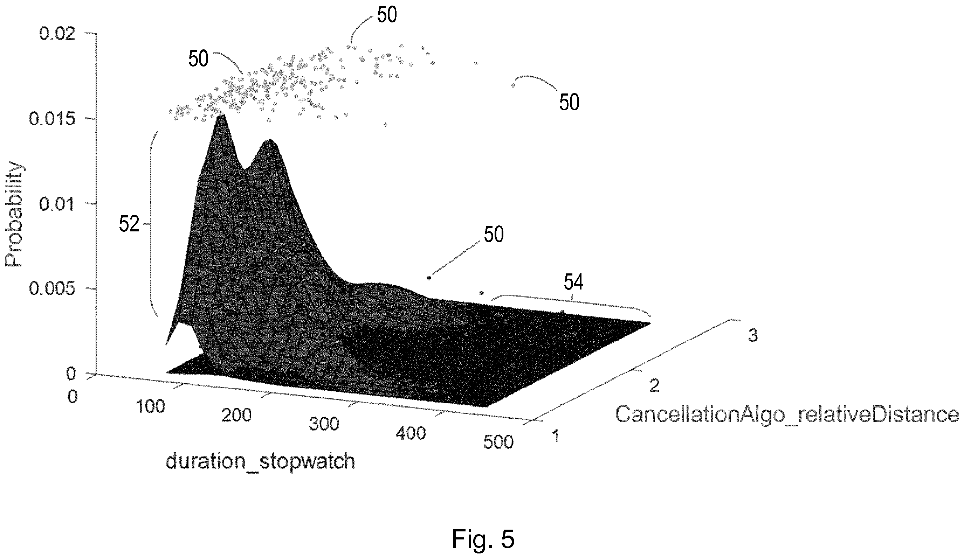

In the example shown in Fig. 4, the test results for all subjects in the population (in this case healthy subjects and those that have suffered a stroke) have been considered together as one dataset, and the probability distribution was formed from that dataset. In an alternative embodiment, the population test results can be separated into subsets based on the medical condition of the subjects (so healthy and suffered a stroke in this example), and a separate probability distribution can be estimated for each subset of population test results. These separate probability distributions can be combined (e.g. summed) to form an overall probability distribution. Optionally, one or more of the probability distributions can be scaled or normalized before combining it with the other probability distributions to take into account the relative occurrence of the relevant medical condition amongst the general population. To this end, one or more probability distributions may be scaled relative to the prevalence of the relevant medical condition so that the overall probability distribution appropriately reflects the prevalences for the general population. For example, 50% of the general population may be classified as healthy and 1% has suffered a stroke, and these prevalences of 50% and 1% can be used as weights. An exemplary probability distribution formed by combining separate and scaled sub-probability distributions is shown in Fig. 5. In Fig. 5, the lighter part of the distribution (generally denoted 52) represents the population test results for the healthy subjects, and the darker part of the distribution (generally denoted 54) represents the population test results for subjects who have suffered a stroke.

An advantage of forming a separate probability distribution for each group of subjects is that the probability distributions can also be used to classify subjects with an unknown health status (e.g. healthy, stroke, Alzheimer's, etc.) into one of the groups of subjects by comparing the test results for that subject to each probability distribution to find the probability distribution that yields the highest probability for those test results.

As an alternative to the above approaches, it is possible to better model the behavior of the probability distribution in the regions where the associated probability is quite low or zero by forming a set of novelty scores from the MPD. In particular embodiments, Extreme Value Theory (EVT), for example as described in "Novelty Detection with Multivariate Extreme Value Statistics" by Clifton, D.A., Hugueny, S. and Tarassenko, L., Journal of Signal Processing Systems 2011 6593, 371-389 is used to better model the behavior of the probability distribution in the regions where the probability is low. Fig. 5 shows a graph of novelty scores derived from the population test data using EVT. It can be seen that the lower the novelty score, the more likely that that test result would have occurred in the population test results, and thus the more likely that that test result is genuine. On the other hand, the higher the novelty score, the more likely that that test result did not occur in the population test results, and thus the more likely that that test result is due to malingering. As with the other probability distributions, a threshold (the second threshold) can be used to identify whether a particular novelty score is indicative of malingering.

There is therefore provided an apparatus and method for detecting malingering in physical and/or mental function tests by a first subject. The apparatus and method enable test results for multiple tests to be assessed together, and to be assessed more easily to detect a malingerer. In particular, using multivariate probability distributions that include parameters from multiple different physical and/or mental function tests means that inter-test relationships are implicitly taken into account in the distribution, and thus across-test relationships represented in the population test results can be exploited. In addition, a further advantage is that malingerers can be detected without the population test results including examples of malingering.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. An apparatus (2) for use in detecting malingering by a first subject in one or more physical and/or mental function tests, the apparatus (2) comprising:
a memory unit (10) configured to store information relating to a multivariate probability distribution, MPD, for a plurality of parameters, wherein each parameter relates to a particular physical and/or mental function test in a set of physical and/or mental function tests, wherein the MPD is determined from one or more population test results, wherein each population test result relates to the performance of one or more tests in the set by a respective subject in a population of subjects and comprises values for the parameters relating to the one or more tests performed by the subject;
a processing unit (8) configured to:
receive a first test result for the first subject relating to the performance of one or more of the tests in the set by the first subject, wherein the first test result comprises values for the parameters relating to the one or more tests performed by the first subject;
determine an indication of whether the first subject was malingering by comparing the values for the parameters in the first test result to the MPD; and
output the determined indication.

2. An apparatus (2) as claimed in claim 1, wherein the processing unit (8) is configured to determine an indication of whether the first subject was malingering by:
determining a probability value from the MPD corresponding to the values for the parameters in the first test result, wherein the probability value indicates a likelihood of observing the first test result in the population of subjects.

3. An apparatus (2) as claimed in claim 2, wherein the processing unit (8) is configured to determine the indication of whether the first subject was malingering by:
comparing the determined probability value to a first threshold; and
determining an indication that the first subject was malingering if the determined probability value is below the first threshold.

4. An apparatus (2) as claimed in any of claims 1-3, wherein the stored information relating to the MDF is a set of novelty scores for the plurality of parameters determined from the MPD, and wherein the processing unit (8) is configured to determine an indication of whether the first subject was malingering by:
determining a novelty score from the set of novelty scores corresponding to the values for the parameters in the first test result.

5. An apparatus (2) as claimed in claim 4, wherein the processing unit (8) is configured to determine the indication of whether the first subject was malingering by:
comparing the determined novelty score to a second threshold; and
determining an indication that the first subject was malingering if the determined novelty score is above the second threshold.

6. An apparatus (2) as claimed in any of claims 1-5, wherein the processing unit (8) is further configured to:
receive the one or more population test results; and
determine the MPD from the one or more population test results.

7. An apparatus (2) as claimed in claim 6, wherein the processing unit (8) is configured to determine the MPD from the one or more population test results by:
forming an N-dimensional histogram from the population test results, where N is the number of parameters in the plurality of parameters.

8. An apparatus (2) as claimed in claim 6, wherein the processing unit (8) is configured to determine the MPD from the one or more population test results using a mixture model.

9. An apparatus (2) as claimed in any of claims 6-8, wherein the processing unit (8) is configured to determine the MPD from the one or more population test results by:
determining a plurality of sub-MPDs from respective subsets of the population test results, wherein each subset of population test results comprises population test results for subjects having a similar health status; and
combining the plurality of sub-MPDs to form the MPD.

10. A computer-implemented method for detecting malingering by a first subject in one or more physical and/or mental function tests, the method comprising:
storing (101), in a memory unit, information relating to a multivariate probability distribution, MPD, for a plurality of parameters, wherein each parameter relates to a particular physical and/or mental function test in a set of physical and/or mental function tests, wherein the MPD is determined from one or more population test results, wherein each population test result relates to the performance of one or more tests in the set by a respective subject in a population of subjects and comprises values for the parameters relating to the one or more tests performed by the subject;
receiving (103), at a processing unit, a first test result for the first subject relating to the performance of one or more of the tests in the set by the first subject, wherein the first test result comprises values for the parameters relating to the one or more tests performed by the first subject;
determining (105), by the processing unit, an indication of whether the first subject was malingering by comparing the values for the parameters in the first test result to the MPD; and
outputting (107), by the processing unit, the determined indication.

11. A method as claimed in claim 10, wherein the step of determining an indication of whether the first subject was malingering comprises:
determining a probability value from the MPD corresponding to the values for the parameters in the first test result, wherein the probability value indicates a likelihood of observing the first test result in the population of subjects.

12. A method as claimed in claim 11, wherein the step of determining the indication of whether the first subject was malingering comprises:
comparing the determined probability value to a first threshold; and
determining an indication that the first subject was malingering if the determined probability value is below the first threshold.

13. A method as claimed in any of claims 10-12, wherein the stored information relating to the MDF is a set of novelty scores for the plurality of parameters determined from the MPD, and wherein the step of determining an indication of whether the first subject was malingering comprises:
determining a novelty score from the set of novelty scores corresponding to the values for the parameters in the first test result.

14. A method as claimed in claim 13, wherein the step of determining the indication of whether the first subject was malingering comprises:
comparing the determined novelty score to a second threshold; and
determining an indication that the first subject was malingering if the determined novelty score is above the second threshold.

15. A computer program product comprising a computer readable medium having computer readable code embodied therein, the computer readable code being configured such that, on execution by a suitable computer or processor, the computer or processor is caused to perform the method of any of claims 10-14.
